# EUROPEAN PATENT APPLICATION

(11) **EP 4 495 253 A1**
(43) Date of publication of application: **22.01.2025**
(21) Application number: 23770489.5
(22) Date of filing: 06.03.2023
(51) Int. Cl.: C12Q 1/686, C12M 1/00, C12M 1/34

(54) **TESTING SUPPORT DEVICE**

(30) Priority: 16.03.2022 JP 2022041122
(71) Applicant: KAWASAKI JUKOGYO KABUSHIKI KAISHA, Kobe-shi, Hyogo 650-8670 (JP)
(72) Inventor: TSUCHIYA, Hayato, Kobe-Shi, Hyogo 650-8670 (JP); YAMAGUCHI, Jun, Kobe-Shi, Hyogo 650-8670 (JP)
(74) Representative: Hoffmann Eitle
(86) International application number: PCT/JP2023/008212
(87) International publication number: WO 2023/176532

(57) **Abstract**

The testing support device comprises a communicator and a processor. The communicator receives amplification date for testing that shows the degree of amplification of a first gene derived from a virus or a bacterium to be tested and the degree of amplification of a second gene derived from a subject, obtained by subjecting a sample to PCR. The processor determines a score that shows the degree to which the amplification data for testing deviate from standard amplification data using a model constructed by machine learning a plurality of amplification data for learning, and outputs the score.

## Description

### TECHNICAL FIELD

The present application mainly relates to a testing support device that supports testing based on amplification data obtained by a PCR method.

### BACKGROUND ART

PTL 1 discloses an electrocardiogram display apparatus. The electrocardiogram display apparatus inputs divided electrocardiogram data, which is obtained by dividing into electrocardiogram data having a predetermined time length, into a machine learning model. The machine learning model is created by machine learning using a plurality of pieces of supervised electrocardiogram data. The machine learning model extracts and outputs waveform portions where heart disease is suspected, from the divided electrocardiogram data.

### PATENT LITERATURE

Patent Literature 1: Japanese Patent Application Laid-Open No. 2021-106902

### SUMMARY

### TECHINCAL PROBLEM

In the method of PTL 1, a portion where the probability of heart disease is estimated to be extremely high and a portion where the probability of heart disease is estimated to be not very high are indicated in the same manner. Thus, a doctor who obtains the waveform portion outputted by machine learning cannot recognize whether the waveform portion clearly indicates heart disease or whether the waveform portion indicates a slight possibility of heart disease, and therefore, it may take time to perform determination regarding the waveform portion. This type of problem is not limited to testing for heart disease, but is a common problem in various tests.

The present application has been made in view of the circumstances described above, its main object is to provide, in a testing support device that supports testing using a PCR method, a configuration that facilitates determination based on data obtained by testing.

### SOLUTION TO PROBLEM

Problems to be solved by the present disclosure are as described above, and next, means for solving the problems and effects thereof will be described.

According to an aspect of the present disclosure, a testing support device having the following configuration is provided. That is, the testing support device includes a communicator and a processor. The communicator receives testing amplification data indicating a degree of amplification of a first gene derived from a virus or a bacterium to be tested and a degree of amplification of a second gene derived from a subject. The processor uses a model to obtain and output a score indicating a degree to which the testing amplification data deviates from standard amplification data, the model being constructed by using machine learning to learn a plurality of learning amplification data.

### ADVANTAGEOUS EFFECTS

According to the present application, in a testing support device that supports testing by using a PCR method, determination based on data obtained by testing can be easily performed.

### BRIEF DESCRIPTION OF THE DRAWINGS

FIG. 1 is a block diagram of a testing support system.
FIG. 2 is a graph illustrating a standard amplification curve waveform when a sample is negative.
FIG. 3 is an explanatory diagram illustrating a first example of a process performed by the testing support device.
FIG. 4 is an explanatory diagram illustrating a second example of a process performed by the testing support device.
FIG. 5 is a graph illustrating an example in which a waveform is deviated from the standard amplification curve waveform when a sample is negative.
FIG. 6 is a graph illustrating another example in which a waveform is deviated from the standard amplification curve waveform when a sample is negative.
FIG. 7 is an explanatory diagram illustrating a third example of a process performed by the testing support device.
FIG. 8 is a screen example of a display.
FIG. 9 is a block diagram illustrating an example in which the testing support system is provided in a testing center.

### EMBODIMENT

Next, an embodiment of the present application will be described with reference to drawings.

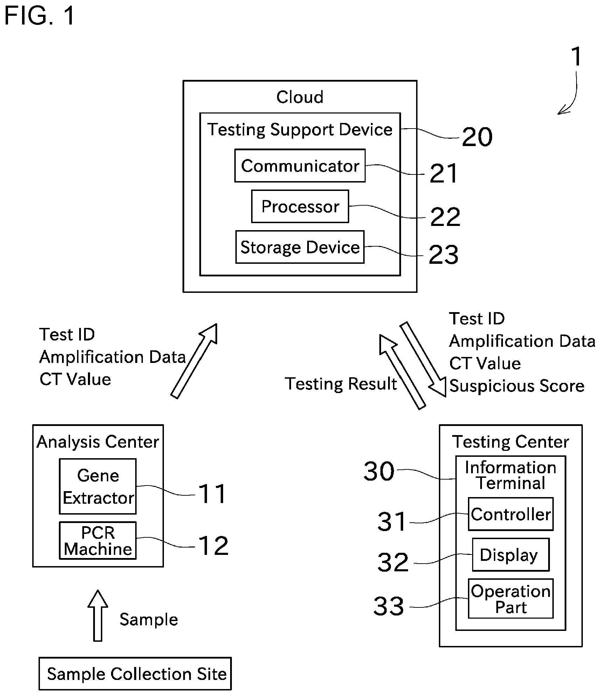

A testing support system 1 illustrated in FIG. 1 is a system for supporting testing to determine whether a person has an infection using a PCR method. In the following, this type of testing is simply referred to as "testing". As illustrated in FIG. 1, the testing support system 1 includes devices placed at a sample collection site, a cloud, and an analysis center, respectively. The devices included in the testing support system 1 may be placed at the same location, as described below.

An infection to be tested of the present embodiment is COVID-19, and a virus that causes COVID-19 is SARS-CoV-2. COVID-19 is also referred to as novel coronavirus infection. However, the testing support system 1 can also be applied to a virus or a bacteria causing infections other than COVID-19. Specifically, the testing support system 1 can also be applied to Mycobacterium tuberculosis or pneumonia virus. In the following, a virus causing an infection to be tested will be simply referred to as a virus.

In the sample collection site, a sample for testing is collected from a subject. The sample is, for example, saliva or nasal swab of the subject, but can also be other body fluids. The sample collected at the sample collection site is transported to the analysis center.

A gene extractor 11 and a PCR machine 12 are provided in the analysis center. The gene extractor 11 extracts gene from the sample. The PCR machine 12 performs the PCR method, so that the gene extracted from the sample is amplified to determine the degree of amplification.

Analysis at the analysis center is performed by a test assistant operating the gene extractor 11 and the PCR machine 12. However, instead of the test assistant, an industrial robot such as an arm robot may perform the analysis. The gene extractor 11 and the PCR machine 12 may be provided at the sample collection site or the testing site.

The PCR method has been known, and will be therefore simply described. The PCR method includes a thermal displacement step, an annealing step, and an extension step. In the thermal displacement step, the sample is heated to separate double-stranded DNA and transform the double-stranded DNA into two single-stranded DNA. In the annealing step, the temperature is lowered to cause primer to bind to each single-stranded DNA. In the extension step, the temperature is raised again to extend the primer, which generates double-stranded DNA. This completes one cycle of the process. By performing one cycle of the process, the number of DNA can be doubled. By repeating this cycle of the process, the number of DNA can be greatly amplified. When DNA is amplified, fluorescence is generated. Therefore, a fluorescence intensity for each cycle is measured to calculate the degree of amplification of a gene.

For example, when the sample contains a virus, the virus is amplified by the PCR method. Therefore, whether the sample contains a virus can be detected based on presence or absence of an increase in the fluorescence intensity of the virus. The sample contains not only gene derived from the virus but also gene derived from the subject certainly. Accordingly, the gene derived from the subject is amplified by the PCR method. Thus, when the testing is performed appropriately, the fluorescence intensity of the gene derived from the subject increases in association with a progress of the number of cycles.

The PCR method is an analytical method for DNA. However, SARS-CoV-2 is RNA. Thus, the above-mentioned PCR method is performed after converting RNA to cDNA using reverse transcriptase. This type of PCR method is referred to as an RT-PCR method. In this specification, the RT-PCR method and the general PCR method without reverse transcription are collectively referred to as the PCR method.

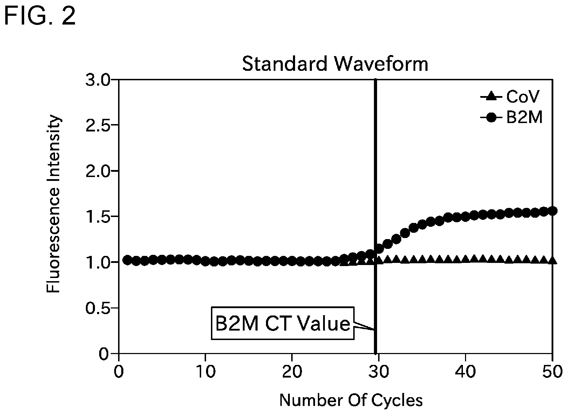

FIG. 2 illustrates a graph plotting results obtained by the PCR method. A horizontal axis is the number of cycles, and a vertical axis is the fluorescence intensity. A triangular marker with CoV is a plot indicating the fluorescence intensity of the virus. A circular marker with B2M is a plot indicating the fluorescence intensity of B2M gene derived from the subject. An official name of the B2M gene is β2-microglobulin gene. Since this sample does not contain a virus, the fluorescence intensity of the virus hardly changes even when the number of cycles increases.

On the other hand, naturally, the sample contains the B2M gene. The fluorescence intensity remains below a lower limit of detection until the number of B2M gene reaches a certain number, and therefore, the fluorescence intensity does not change. After that, as the number of cycles increases, the fluorescence intensity is higher than the lower limit of detection, and then increases in the graph. A B2M CT value is the number of cycles at a point when an increase in the fluorescence intensity of the B2M gene is detected. When the sample contains the virus, the fluorescence intensity of the virus increases in association with the increase in the number of cycles. Thus, a CoV CT value indicating such increase can be calculated. The CT value of the virus and the CT value of the B2M gene are collectively referred to simply as the CT value.

The change of the fluorescence intensity depending on the number of cycles indicates the degree of gene amplification by the PCR method. Therefore, in the following, data indicating the change of the fluorescence intensity depending on the number of cycles is referred to as amplification data. The amplification data is numerical data that associates the number of cycles with the fluorescence intensity. FIG. 2 illustrates a waveform created based on such numerical data. The waveform illustrated in FIG. 2 can be treated as image data, but in this case, it is necessary to convert the waveform from the image data to the numerical data before using.

The PCR machine 12 and the testing support device 20 can communicate with each other via a wide area network. The wide area network is, for example, the Internet, but may be network other than the Internet. An example of the network other than the Internet is a network that connects local area networks at different locations with a dedicated line. The PCR machine 12 transmits the amplification data and the CT value to the testing support device 20 in association with a test ID for identifying the sample or testing. A computer that receives the amplification data, and the like from the PCR machine 12 may be provided, and the computer may transmit the amplification data, and the like to the testing support device 20.

The testing support device 20 is provided in a facility or a data center of an administrator of the testing support system 1. The testing support device 20 provides services to the analysis center and the testing center via the wide area network. Therefore, the testing support device 20 can be considered to be provided on the cloud. The testing support device 20 is specifically a server and includes a communicator 21, a processor 22, and a storage device 23.

The communicator 21 is a communication module for wired or wireless communication. The communicator 21 receives the amplification data and the like the PCR machine 12 and transmits the amplification data and the like to an information terminal 30 in the testing center. The processor 22 is, for example, a CPU, and performs calculation and control. The processor 22 performs various processes by executing programs stored in the storage device 23. The storage device 23 is, for example, an HDD or SSD, and stores various programs and data.

The testing support device 20 calculates information (suspicious score which will be described later) that is useful for a doctor or a laboratory technician to perform determination based on the amplification data and the CT value received from the PCR machine 12. The testing support device 20 and the information terminal 30 can communicate with each other via the wide area network. The testing support device 20 transmits the amplification data, the CT value, and the suspicious score to the information terminal 30 in association with the test ID.

The laboratory technician is enrolled in the testing center. The laboratory technician determines that the subject is positive or negative, based on the amplification data, the CT value, and the suspicious score. The laboratory technician determines that retesting is required when testing has not been performed appropriately. Instead of the laboratory technician, a doctor may determine whether the subject is positive or negative, or whether retesting is required.

The information terminal 30 is provided in the testing center. The information terminal 30 is a PC or a tablet terminal used by the laboratory technician. The information terminal 30 includes a controller 31, a display 32, and an operation part 33. The controller 31 includes a CPU, an HDD, and the like. The controller 31 performs various processes by the CPU executing programs stored in the HDD, and the like. The display 32 is a display capable of displaying information. The operation part 33 is a keyboard, a mouse, a touch panel, and the like, which are operated by the laboratory technician.

Next, determination performed by the laboratory technician will be briefly described. When there is no increase in the fluorescence intensity of the virus in the amplification data, in other words, when there is no CT value for the virus, the virus is not present in the sample and the result is therefore considered to be negative. In the following, an amplification curve waveform when there is no CT value of the virus is sometimes referred to as a negative waveform. Even when the negative waveform is detected, there may be no CT value of the virus due to the testing not being performed appropriately. Thus, the laboratory technician needs to check the negative waveform to determine whether the result is negative or retesting is required. For example, when the B2M gene waveform of the negative waveform deviates from the standard waveform, there is a possibility that the testing has not been performed appropriately. That is, the laboratory technician needs to monitor and check all negative waveforms without the CT value of the virus.

In reality, the negative waveforms include "obvious negative waveform" and "suspicious negative waveform". Since the obvious negative waveform has a high degree of coincidence with the standard negative waveform, the laboratory technician can appropriately determine by simply checking the waveform. On the other hand, since the suspicious negative waveform deviates significantly from the standard negative waveform, the laboratory technician needs to check the waveform with sufficient attention. However, there are many types of suspicious negative waveforms, and therefore, it is difficult for machines to automatically determine such suspicious negative waveforms.

In the present embodiment, the testing support device 20 uses machine learning to obtain a score that indicates the degree of deviation from the standard waveform. Such score is an indicator indicating the degree of suspicion of the negative result, and thus, hereinafter referred to as a suspicious score. The laboratory technician checks the suspicious score to recognize whether the waveform to be determined needs to be checked with attention. As a result, the burden on the laboratory technician can be reduced.

Even when the sample contains the virus and when the fluorescence intensity of the virus increases with the CT value of the virus, there is a possibility that the testing has not been performed appropriately. Thus, the laboratory technician needs to check the waveform of the amplification curve to determine whether the sample indicates a positive result or retesting is required. However, the number of samples indicating positive results is generally small. Thus, determination of whether the sample indicates the positive result or whether retesting is required is not a significant burden on the laboratory technician. Therefore, in the present embodiment, the suspicious score is not calculated for the positive waveform. The suspicious score may be calculated for the positive waveform in a situation where there are many positive samples, for example.

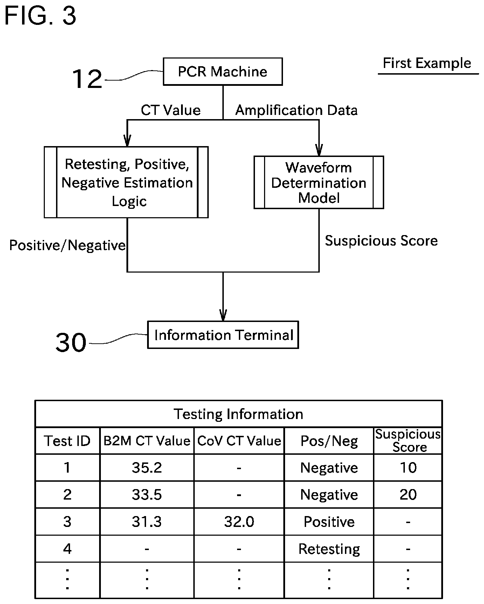

Next, a process for the CT value and the amplification data performed by the testing support device 20 will be described. In the following, three examples illustrated in FIG. 3, FIG. 4, and FIG. 7 will be described. The examples use different learning models for calculating the suspicious score, respectively.

Firstly, a first example will be described with reference to FIG. 3. FIG. 3 illustrates an estimation logic for positive, negative, or retesting, and a waveform determination model. The estimation logic and the waveform determination model are processes performed by the testing support device 20.

In the estimation logic, estimation is performed whether the sample is positive or negative, or retesting is required, based on the CT value received from the PCR machine 12. The estimation by the estimation logic is intended to assist a laboratory technician, and a final determination is made by the laboratory technician. In the estimation logic, the estimation is performed based on a pre-created rule, not on a model constructed by machine learning. In the estimation logic, it is determined whether retesting is required, based on the CT value of the B2M gene. That is, in the estimation logic, it is determined that retesting is required when the CT value of the B2M gene is not present or when a threshold condition is not met even in the presence of the CT value of the B2M gene. The threshold condition of the CT value of the B2M gene is determined experimentally or empirically, for example.

In the estimation logic, the result is determined to be negative when the CT value of the B2M gene meets the threshold condition and there is no CT value of the virus. In the estimation logic, the result is determined to be positive when the CT value of the B2M gene meets the threshold condition and the CT value of the virus meets the threshold condition. The threshold condition of the CT value of the virus is determined experimentally or empirically, for example. The estimation by the estimation logic is intended to assist a laboratory technician for determination, and is therefore not a necessary process. Such estimation by the estimation logic may be omitted.

In the waveform determination model, the suspicious score is outputted by inputting the amplification data received from the PCR machine 12. The waveform determination model is a model constructed by supervised machine learning, for example. Specifically, a plurality of learning amplification data indicating standard negative waveforms and learning amplification data indicating negative waveforms that is determined to require retesting are prepared, and these amplification data are inputted together with correct answers (that is, the standard negative waveform or the negative waveform that deviates from the standard waveform and is determined to require retesting) for learning. Accordingly, trends and features indicated by the standard negative waveform are learned, and the waveform determination model is constructed. Alternatively, the learning amplification data indicating the standard negative waveform and the learning amplification data indicating the negative waveform that deviates from the standard negative waveform may be learned by unsupervised learning. Any machine learning method may be used, and a support vector machine may be used, for example. The above-described learning constructs a model in which the amplification data is taken as input and the degree of deviation of the amplification data from the standard negative waveform is outputted as the suspicious score. Since this type of model constructing method has been already known, detailed construction process will be omitted.

The amplification data to be trained is both the amplification data of the B2M gene and the amplification data of the virus. The amplification data of the B2M gene and the amplification data of the virus are trained together to construct a single model.

As described above, the amplification data to be trained is in a numerical format. In addition, feature amount extracted from the amplification data of the standard negative waveform or the negative waveform determined to require retesting may be trained. For example, when the waveform of the learning amplification data approximates a specified function, a coefficient of a maximum likelihood function of the specified function or the amount of deviation from the maximum likelihood function can be used as the feature amount. The specified function is, for example, a sigmoid function, but a different function may also be used depending on the waveform of the learning amplification data.

The above-described process constructs the waveform determination model. At a time of determination, testing amplification data received from the PCR machine 12 is inputted to the waveform determination model. In the waveform determination model, whether or not the testing amplification data can be classified into the standard negative waveform (in other words, whether it is classified into a waveform that deviates from the standard negative waveform) is outputted as a determination result. Furthermore, in the waveform determination model, the degree of deviation from the standard negative waveform is outputted as the suspicious score. As described above, in the waveform determination model, the features of the standard negative waveforms are learned, so that the suspicious score can be outputted based on the degree of deviation from such features. When the waveform determination model by learning the feature amount is constructed, the feature amount for testing is extracted based on the testing amplification data. The extracted feature amount for testing is then inputted to the waveform determination model.

In the waveform determination model, whether the testing amplification data is classified into a group of the standard negative waveforms and to what extent are outputted. Therefore, it can be realized by unsupervised learning. For example, for a group of the standard negative waveforms, the suspicious score can be calculated based on whether the testing amplification data belongs to the same group and the degree of discrepancy.

As described above, in the waveform determination model, the suspicious score for the testing amplification data received from the PCR machine 12 is outputted. The testing support device 20 adds an estimation result of the estimation logic and the suspicious score outputted by the waveform determination model to the CT value and the amplification data received from the PCR machine 12, and transmits them to the information terminal 30. The laboratory technician determines the waveform more carefully for a sample with a high suspicious score since a possibility of retesting is higher than for other samples. On the other hand, the laboratory technician can determine the waveform easily for a sample with a low suspicious score since a possibility of retesting is lower than for other samples.

The information terminal 30 may transmit the determination result by the laboratory technician to the testing support device 20. The testing support device 20 may use the determination result by the laboratory technician received from the information terminal 30 as incremental learning data. Specifically, the determination result by the laboratory technician received from the information terminal 30 and the amplification data used in the determination are used to perform supervised incremental learning for the waveform determination model, so that the waveform determination model can be updated. This can obtain a more accurate suspicious score.

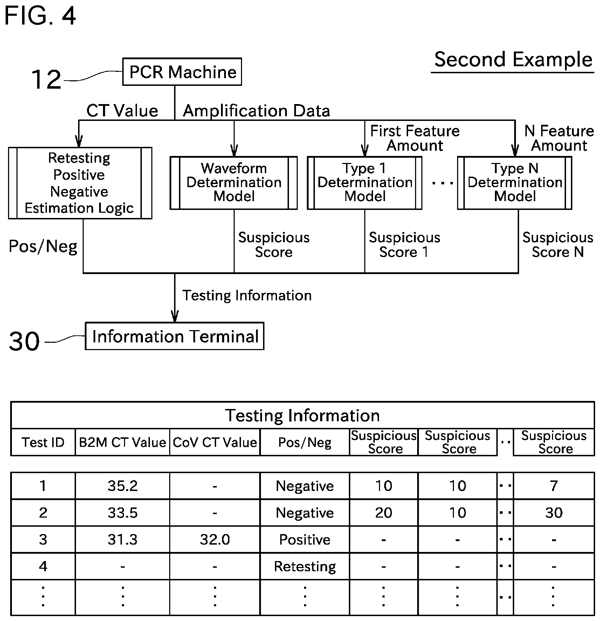

Next, a second example illustrated in FIG. 4 will be described. FIG. 4 illustrates the estimation logic for positive, negative, or retesting, the waveform determination model, and a plurality of type determination models. Since the estimation logic and the waveform determination model of the second example are the same as those of the first example, description thereof is omitted. Thus, in the following, the type determination model will be described. The waveform determination model can be omitted from the second example.

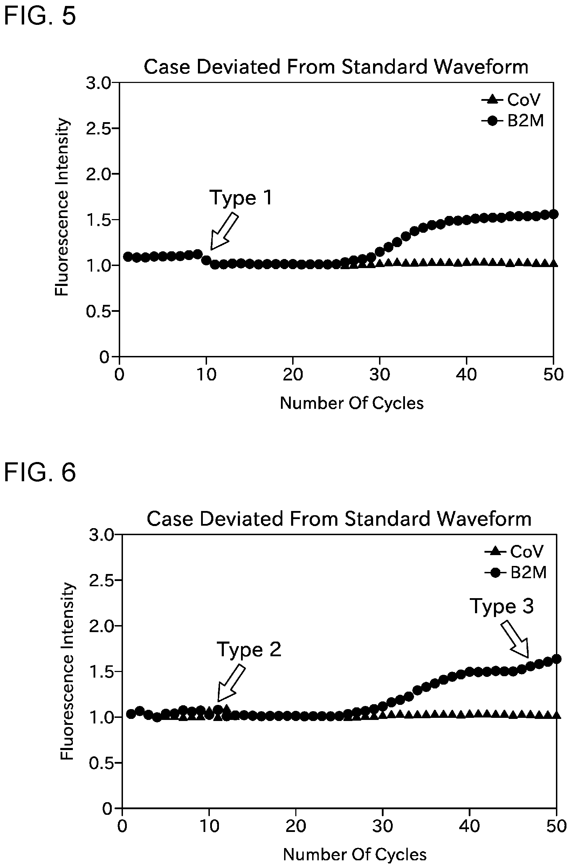

The laboratory technician determines the waveform from a plurality of viewpoints. Specifically, there are several types of waveforms that deviate from the standard waveform. FIG. 5 illustrates Type 1. Type 1 indicates a sudden decrease in the fluorescence intensities of the B2M gene and the virus in a range smaller than the CT value. FIG. 6 illustrates Type 2 and Type 3. Type 2 indicates that the fluorescence intensities of the B2M gene and the virus fluctuate greatly and are not stable in a range smaller than the CT value. Type 3 indicates that the fluorescence intensity of the B2M gene increases again after exceeding the CT value and no further changes are observed. These types are examples, and there may be various types.

The type determination model is a model constructed individually for each type in order to approximate the determination by the laboratory technician. The plurality of type determination models correspond to individual models. For example, the type determination model of Type 1 learns a plurality of waveforms, including the standard waveform as well as waveforms that deviate from the standard waveform. As a result, the more the testing amplification data deviates from the standard waveform as illustrated in FIG. 5, the higher the suspicious score becomes. The detailed learning method and changeable points are the same as those of the unsupervised learning model of the waveform determination model in the first example. That is, "the learning amplification data indicating the negative waveform determined that retesting is required" in the first example is replaced with "the learning amplification data in which Type 1 appears" to construct a model. Such learning in the above-described manner constructs the type determination model that outputs the degree of deviation of the amplification data from the negative waveform as the suspicious score using the amplification data taken as an input, and outputs a higher suspicious score especially as the behavior is closer to Type 1. In other words, from the viewpoint of Type 1, the type determination model that outputs the suspicious score indicating the degree of deviation from the standard negative waveform is constructed. The feature amount indicating whether to include Type 1 may be extracted from the amplification data in the same way as in the first example, and such feature amount may be learned. For example, in a function created to indicate Type 1, a coefficient of a maximum likelihood function or the amount of deviation from the maximum likelihood function, in other words, the amount indicating the degree of conformity or non-conformity to Type 1, can be used as the feature amount in place of the "learning amplification data indicating the negative waveforms determined to require retesting". Similarly, for Type 2 and Type 3 illustrated in FIG. 6, learning is performed individually to construct respective type determination models. In the type determination model of Type 2, the more the fluorescence intensities of the B2M gene and the virus greatly fluctuate within a range smaller than the CT value, the higher the suspicious score is outputted, as illustrated in FIG. 6. In the type determination model of Type 3, the more the fluorescence intensity of the B2M gene increases again after the fluorescence intensity exceeds the CT value and no further changes are observed, the higher the suspicious score is outputted, as illustrated in FIG. 6. The above-described process is performed for N viewpoints, so that N-type determination model is constructed.

In the second example, in addition to the estimation result of the estimation logic and the suspicious score of the waveform determination model, suspicious scores 1 to N for each type determination model is transmitted to the information terminal 30. The laboratory technician sees the suspicious score of the waveform determination model to determine whether the likelihood of retesting is high when considering the entire waveform. In other words, the laboratory technician sees the suspicious score of the waveform determination model to obtain comprehensive evaluation. The laboratory technician also sees the suspicious scores 1 to N of an individual determination model to obtain evaluation for each viewpoint. For example, when the suspicious score 1 is high, the laboratory technician pays particular attention to Type 1 to perform appropriate determination.

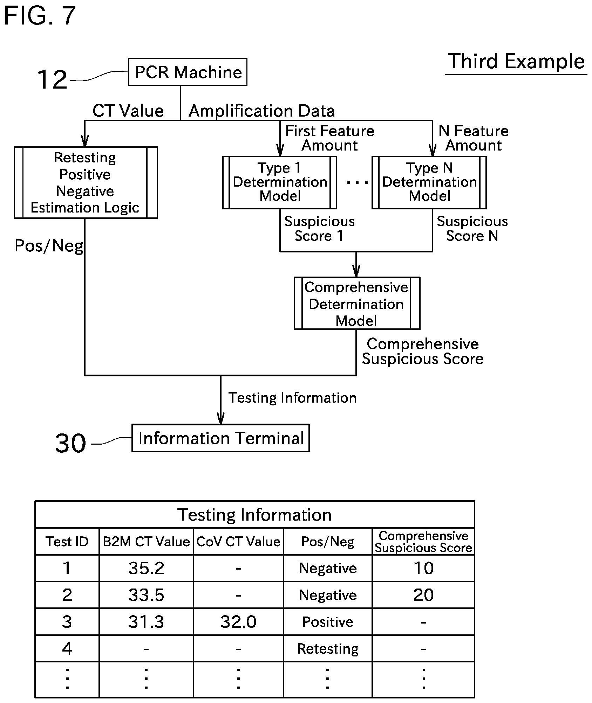

Next, a third example illustrated in FIG. 7 will be described. FIG. 7 illustrates an estimation logic for positive, negative, or retesting, a plurality of type determination models, and a comprehensive determination model. The estimation logic of the third example is the same as that of the first example, and the type determination models of the third example are the same as those of the second example. Thus, the description thereof will be omitted.

The laboratory technician determines the waveform from a plurality of viewpoints and then reaches a conclusion by comprehensively considering the determination result by the plurality of viewpoints. For example, the laboratory technician reaches a conclusion by considering the degree of deviations for each type and the combination of types that have occurred.

The comprehensive determination model of the third example imitates the comprehensive determination by the laboratory technician. The comprehensive determination model is constructed by using the suspicious scores outputted by the plurality of type determination models respectively, and the determination results by the laboratory technician based on such suspicious scores, as learning targets. The suspicious scores of the plurality of type determination models are inputted to such constructed comprehensive determination model, so that a determination logic of the laboratory technician is imitated to obtain how close the result is to either negative or retesting. Then, the comprehensive determination model generates and outputs a comprehensive suspicious score such that the closer to a negative result, the lower the comprehensive suspicious score because of low suspicion, and such that the closer to retesting, the higher the comprehensive suspicious score because of high suspicion.

**In** the third example, the estimation result of the estimation logic and the comprehensive suspicious score of the comprehensive determination model are calculated and transmitted to the information terminal 30. The laboratory technician sees the comprehensive suspicious score to easily determine whether there is a high possibility of retesting. **In** particular, since a method of calculating the comprehensive suspicious score in the third example imitates the determination method by the laboratory technician, the laboratory technician can receive a highly reliable comprehensive suspicious score.

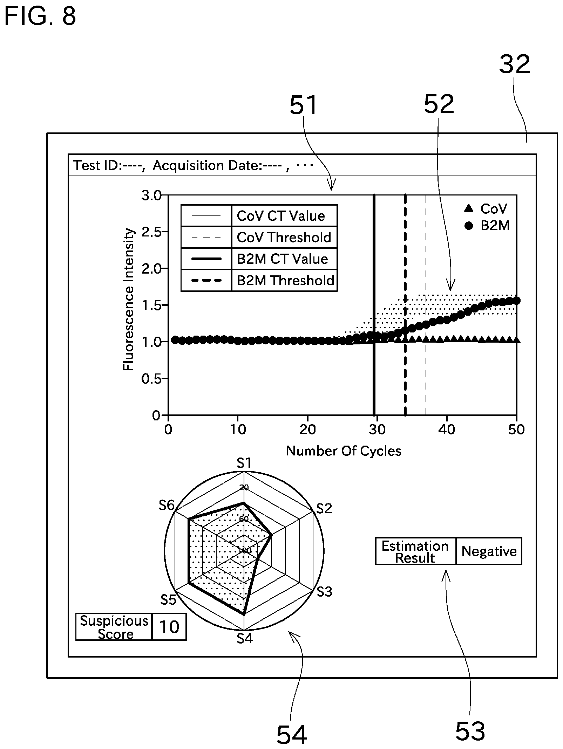

Next, a screen example in which the suspicious score and the like calculated by the testing support device 20 are displayed on the display 32 of the information terminal 30 will be described with reference to FIG. 8. FIG. 8 illustrates the suspicious score calculated by performing the process of the above-described second example.

As illustrated in FIG. 8, the display 32 displays thereon an amplification curve 51, a standard waveform range 52, an estimation result box 53, and a radar chart 54, for example.

The amplification curve 51 is a graph indicating a change in the fluorescence intensities of the B2M gene and the virus as described above. The amplification curve 51 indicates the CT value of the B2M gene, its threshold, the CT value of the virus, and its threshold. Since a sample of the example illustrated in FIG. 8 is negative, the CT value of the virus is not indicated.

The standard waveform range 52 is a range of the standard waveform of the amplification curve 51 of the B2M gene. The standard waveform range 52 displayed to be superimposed on the amplification curve 51. This easily determines whether the amplification curve 51 is included in the standard waveform range 52. The standard waveform range 52 can be generated, for example, by aggregating the learning amplification data. The standard waveform range 52 is not necessary, and its indication may be omitted.

The estimation result box 53 displays thereon the estimation result of the estimation logic for positive, negative, or retesting. The estimation result box 53 is not necessary, and its indication may be omitted.

The radar chart 54 indicates the suspicious score. The radar chart 54 displays thereon the suspicious score of each waveform determination model in a numerical format, and displays the suspicious scores 1 to N for each type determination model in a radar chart format. The smaller the suspicious score of the type determination model and the like, the larger the radar chart. That is, the closer the waveform is to the standard waveform, the larger the radar chart is. In the present embodiment, although the suspicious score is displayed using the radar chart, the suspicious score may be displayed using other chart formats, for example, a bar graph. In this specification, the chart format means a format that represents information using a graph, figure, table, and the like. The suspicious scores 1 to N may be displayed in the numerical format, instead of the chart format. In addition to the numerical values of the suspicious score, a display format of the waveform at a portion with a high suspicious score may be changed to highlight such portion. For example, when the suspicious score of Type 1 is high, a display color of the waveform related to Type 1 may be changed or a marker may be added. Instead of or in addition to the change of the display format of the waveform, a portion with the high suspicious score may be displayed in text. For example, a message such as "there may be deviation from the standard waveform in range of the number of cycles 10 to 20." may be displayed.

The laboratory technician checks such indications to determine the amplification curve for a testing subject, considering the degree of suspicion for the estimation result of "negative" indicated in the estimation result box 53.

As described above, the testing support device 20 of the present embodiment includes the communicator 21 and the processor 22. The communicator 21 receives the testing amplification data, which is obtained by performing the PCR method on a sample and which indicates the degree of amplification of a first gene derived from a virus or a bacterium to be tested and the degree of amplification of a second gene derived from a subject. The processor 22 uses a model constructed by using machine learning to learn a plurality of learning amplification data to obtain and output a score indicating the degree to which the testing amplification data deviates from the standard amplification data. The above-described configuration corresponds to Feature 1.

This allows the laboratory technician to refer to the score and perform determination considering the degree to which the amplification data deviates from the standard amplification data.

In the testing support device 20 of the present embodiment, a model is constructed by using machine learning to learn the feature amount extracted from the learning amplification data. The processor 22 extracts the feature amount from the testing amplification data and inputs the feature amount into the model to obtain the score.

This can reduce the amount of data as compared with the learning of the image data such as the waveform.

In the testing support device 20 of the present embodiment, the processor 22 includes a plurality of individual models. Each of the individual models obtains the score of the testing amplification data in a specific type that deviates from the standard amplification data.

Accordingly, the score can be obtained using a method similar to the determination method by the laboratory technician.

In the testing support device 20 of the present embodiment, the processor 22 inputs the testing amplification data to convert the score outputted by each of the individual models into the chart format and output the converted score.

This allows the laboratory technician to intuitively recognize the score.

In the testing support device 20 of the present embodiment, the processor 22 outputs an image indicating an amplification curve created based on the testing amplification data and a range of an amplification curve created based on the standard amplification data.

This allows the laboratory technician to intuitively recognize whether the amplification curve to be determined deviates from the standard range.

In the testing support device 20 of the present embodiment, the processor 22 estimates whether a result is positive, negative, or retesting based on the CT value of the first gene and the CT value of the second gene, and outputs the estimation result of positive, negative, or retesting together with the score.

This allows the laboratory technician to perform determination using the estimation result as well as the score.

In the testing support device 20 of the present embodiment, the communicator 21 receives the testing amplification data from the PCR machine 12 via the wide area network.

This allows the testing support device 20 to process the amplification data received from a remote location.

In the testing support device 20 of the present embodiment, the communicator 21 transmits the testing amplification data and the score to a facility where the laboratory technician determines the testing amplification data.

This allows the testing support device 20 to assist the determination by the laboratory technician at the remote location.

In the testing support device 20 of the present embodiment, the processor 22 updates the model by learning the testing amplification data and the determination result of the testing amplification data determined by the laboratory technician.

This can improve accuracy of the model.

The testing support device 20 of the second example and the third example of the present embodiment has the following Feature 2 in addition to the above-described Feature 1. That is, the processor 22 includes a plurality of individual models each serving as a model in accordance with a specific type deviated from the standard amplification data. The processor 22 uses each individual model to obtain the score of the testing amplification data in accordance with the specific type.

The testing support device 20 of the second example and the third example of the present embodiment has the following Feature 3 in addition to the above-described Features 1 and 2. That is, the individual model is constructed by using machine learning to learn the feature amount that is extracted for each specific type from the learning amplification data and that indicates the degree of applicability or non-applicability to the specific type. The processor 22 extracts the feature amount from the testing amplification data and inputs the feature amount into the individual model to obtain the score.

Although a preferred embodiment of the present disclosure has been described as above, the above-described configuration may be modified as follows, for example.

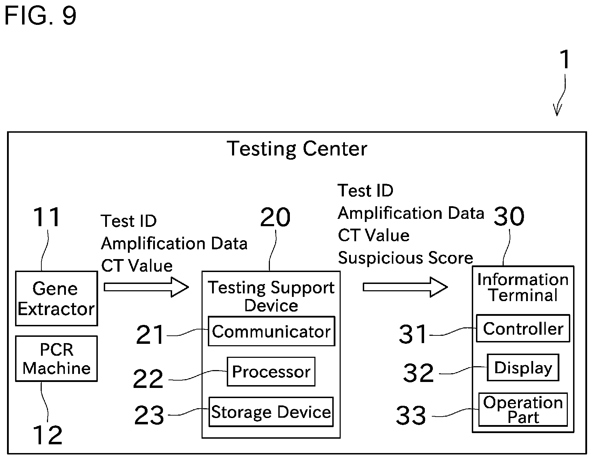

In the above-described embodiment, the testing support device 20 is placed on the cloud, but this is an example. For example, as illustrated in FIG. 9, the testing support device 20 may be placed in a testing center. In this case, the gene extractor 11 and the PCR machine 12 may be provided in the testing center. This allows the testing support system 1 to be provided in the testing center.

The functions of each component disclosed in this application may be performed using a circuit or a processing circuit including a general-purpose processor, a special-purpose processor, an integrated circuit, ASIC (Application Specific Integrated Circuits), a conventional circuit, and/or a combination thereof, configured or programmed to perform the disclosed functions. A processor includes transistors and other circuits, and is therefore considered as the processing circuit or the circuit. In this disclosure, a circuit, a unit, or means is defined as a hardware that executes the recited functions or a hardware that executes the recited functions. The hardware may be a hardware disclosed herein or other known hardware programmed or configured to execute the recited functions. When the hardware is considered to be a processor that is a type of circuit, the circuit, means, or the unit is a combination of the hardware and the software, and the software is used to configure the hardware and/or the processor.

## Claims

1. A testing support device comprising:
a communicator receives testing amplification data which is obtained by performing a PCR method on a sample and which indicates a degree of amplification of a first gene derived from a virus or a bacterium to be tested and a degree of amplification of a second gene derived from a subject; and
a processor uses a model to obtain and output a score indicating a degree to which the testing amplification data deviates from standard amplification data,
the model being constructed by using machine learning to learn a plurality of learning amplification data.

2. The testing support device according to claim 1, wherein
the model is constructed by using machine learning to learn a feature amount extracted from the learning amplification data, and
the processor extracts the feature amount from the testing amplification data and input the feature amount into the model to obtain the score.

3. The testing support device according to claim 1 or 2, wherein
the processor includes a plurality of individual models each serving as the model, and
each of the individual models obtains the score of the testing amplification data in a specific type that deviates from the standard amplification data.

4. The testing support device according to claim 3, wherein
the processor inputs the testing amplification data, to convert the score outputted by each of the individual models into a chart format and output the converted score.

5. The testing support device according to any one of claims 1 to 4, wherein
the processor outputs an image indicating an amplification curve created based on the testing amplification data and a range of an amplification curve created based on the standard amplification data.

6. The testing support device according to any one of claims 1 to 5, wherein
the processor estimates whether a result is positive, negative, or retesting based on a CT value of the first gene and a CT value of the second gene, and outputs an estimation result of positive, negative, or retesting together with the score.

7. The testing support device according to any one of claims 1 to 6, wherein
the testing support device is provided in a testing center in which testing is performed.

8. The testing support device according to any one of claims 1 to 6, wherein
the communicator receives the testing amplification data from a PCR machine which performs analysis using the PCR method, via a wide area network.

9. The testing support device according to any one of claims 1 to 8, wherein
the communicator transmits the testing amplification data and the score to a facility where a laboratory technician determines the testing amplification data.

10. The testing support device according to any one of claims 1 to 9, wherein
the processor updates the model by learning the testing amplification data and a determination result of the testing amplification data determined by the laboratory technician.
